# EUROPEAN PATENT SPECIFICATION

(11) **EP 3 351 626 B1**
(45) Date of publication and mention of the grant of the patent: **07.09.2022**
(21) Application number: 16846668.8
(22) Date of filing: 16.09.2016
(51) Int. Cl.: C12N 5/0735, C12N 5/10, C12N 5/071

(54) **METHOD FOR PRODUCING PANCREATIC BUD CELLS**
VERFAHREN ZUR HERSTELLUNG VON PANKREASDIVERTIKELZELLEN
PROCÉDÉ DE PRODUCTION DE CELLULES DE BOURGEON PANCRÉATIQUE

(30) Priority: 18.09.2015 JP 2015185662
(43) Date of publication of application: 25.07.2018
(73) Proprietor: Kyoto University, Kyoto-shi, Kyoto 606-8501 (JP)
(72) Inventor: OSAFUNE, Kenji, Kyoto-shi Kyoto 606-8501 (JP); TOYODA, Taro, Kyoto-shi Kyoto 606-8501 (JP); KIMURA, Azuma, Kyoto-shi Kyoto 606-8501 (JP)
(74) Representative: Hoffmann Eitle
(86) International application number: PCT/JP2016/077564
(87) International publication number: WO 2017/047797

(56) References cited:
- WO-A1-2015/178431
- WO-A2-2009/070592
- WO-A2-2010/091241
- JP-A- 2012 507 281
- US-A1- 2009 092 586
- KIMURA AZUMA ET AL: "Small molecule AT7867 proliferates PDX1-expressing pancreatic progenitor cells derived from human pluripotent stem cells", STEM CELL RESEARCH, vol. 24, 1 January 2017 (2017-01-01), pages 61-68, XP085230133, ISSN: 1873-5061, DOI: 10.1016/J.SCR.2017.08.010
- TOYODA, T. et al.: "Cell aggregation optimizes the differentiation of human ESCs and iPSCs into pancreatic bud-like progenitor cells", Stem Cell Research, vol. 14, 2015, pages 185-197, XP029204324,
- NOBUAKI SHIRAKI et al.: "Tanosei Kansaibo to reprogramming Gijutsu: iPS Saibo no Naihaiyokei eno Yudo [Endoderrn differentiation of iPS cells]", JAPANESE JOURNAL OF CLINICAL MEDICINE, vol. 73, no. Special Issue 5, 20 June 2015 (2015-06-20), pages 107-114, XP009509012, ISSN: 0047-1852
- HOSOYA, M.: "Preparation of pancreatic beta-cells from human iPS cells with small molecules", Islets, vol. 4, no. 3, 2012, pages 249-252, XP055092446,
- HORI, Y. et al.: "Growth inhibitors promote differentiation of insulin-producing tissue from embryonic stem cells", PNAS, vol. 99, no. 25, 2002, pages 16105-16110, XP002392968,
- MATHEW, S. et al.: "Analysis of alternative signaling pathways of endoderm induction of human embryonic stem cells identifies context specific differences", BMC systems Biology, vol. 6, no. 154, 2012, pages 1-13, XP021137591,
- JARAMILLO, M. et al.: "Potential for Pancreatic Maturation of Differentiating Human Embryonic Stem Cells is Sensitive to the Specific Pathway of Definitive Endoderm Commitment", PLOS ONE, vol. 9, no. 4, 2014, pages 1-14, XP055372566,

## Description

### TECHNICAL FIELD

The present invention relates to a method for generating pancreatic bud cells and a therapeutic agent for treating a pancreatic disease containing the pancreatic bud cells generated by the method. The present application further relates to a method for treating a pancreatic disease by using the pancreatic bud cells.

### BACKGROUND ART

The pancreas functions as an exocrine gland which secretes digestive enzymes such as pancreatic lipase, trypsin, elastase and pancreatic amylase as well as an endocrine gland which secretes pancreatic hormones such as glucagon, insulin, somatostatin and pancreatic polypeptide (PP). Ghrelin has been known as a stomach hormone and in recent years, it has been reported that ghrelin is also secreted by cells in the pancreas. The pancreatic hormones are produced by pancreas islets that are cell cluster composed of four types of cells: α cells, β cells, δ cells and PP cells in the pancreas.

Insulin plays important roles in promoting glucose utilization, protein synthesis and production and storage of neutral fats, lowering blood glucose level as well as maintaining blood glucose within a normal range. Glucagon is a hyperglycemic hormone that functions via hepatic glycogenolysis or gluconeogenesis, and plays an important role in regulating sugar metabolism along with insulin. Somatostatin acts via a somatostatin receptor and suppresses secretion of various hormones such as glucagon and insulin in the pancreas. PP has been known as a satiety factor that is secreted from the cells in the islets of Langerhans in response to food intake, and suppresses the food intake and suppresses body weight gain. Ghrelin stimulates food intake and increases body weight by lowering fat oxidation.

Diabetes is a disease developed by insufficient secretion of insulin or insufficient response to insulin. Once it is developed, the disease is difficult to be cured completely. Diabetes is roughly classified into two types: type 1 diabetes mellitus which is an insulin-dependent diabetes, and type 2 diabetes mellitus which is a non-insulin-dependent diabetes.

Type 2 diabetes mellitus is a chronic disease begins with acquisition of insulin resistance, and is thought to be a type of diabetes whose onset mechanisms involve lifestyle habits such as obesity due to overeating or lack of exercise and stress. Type 2 diabetes mellitus is often developed in the middle-aged and elderly people, and many of patients with diabetes are affected with type 2 diabetes mellitus.

On the other hand, type 1 diabetes mellitus is a disease caused by destruction of β cells (insulin-producing cells) by, for example, autoimmune diseases and viral infections, which obstruct secretion of insulin in the body. Type 1 diabetes mellitus is mainly treated by a symptomatic therapy, i.e. by insulin administration. Pancreas transplantation or pancreatic islet transplantation is also employed so that the blood glucose level which continuously fluctuates in the body of the patient can become automatically controlled and the burden on the patients is reduced. Pancreas or pancreatic islet transplantation can effectively control the blood glucose level of the patient within the normal range. However, sufficient number of the organs that can be used for transplantation is not available. In addition, the patient who received the transplantation needs to take immunosuppressants for entire life to avoid immunorejection to a graft. Immunosuppressants may cause problems of infections or side effects.

A strategy including inducing insulin-producing cell *in vitro* from cells derived from a patient, and transplanting the induced cells into the patient has been proposed and studied. Insulin-producing cells can be obtained, for example, by obtaining cells from the epithelium of the pancreatic duct of a patient and differentiating the cells *in vitro* into insulin-producing cells.

Insulin-producing cells may also be obtained from pluripotent stem cells such as embryonic stem (ES) cells or induced pluripotent stem (iPS) cells by inducing differentiation of the cells into insulin-producing cells with an activin and retinoic acid (RA) (Patent Literature 1, and Non-Patent Literatures 1 to 5). Further, insulin-generating cells may also be generated by introducing PDX1 into pluripotent stem cells and culturing the cells (Patent Literature 2 and Patent Literature 3) as well as by culturing the pluripotent stem cells in the presence of suitably combined small molecule compounds (Patent Literature 4 and Non-Patent Literature 6). However, it has not been reported that any of the *in vitro* generated insulin-producing cells could successfully developed glucose responsiveness in a living body. On the other hand, it has been reported that pancreas precursor cells were generated and the cells secreted insulin depending on the glucose level when transplanted into a living body (Non-Patent Literatures 7 and 8).

### CITATION LIST

### PATENT LITERATURE

Patent Literature 1: Japanese Patent Publication No. 2009-225661
Patent Literature 2: U.S. Patent No. 7534608
Patent Literature 3: Japanese Patent Publication No. 2006-075022
Patent Literature 4: WO 2011/081222

Non Patent Literature 1: E. Kroon et al., Nature Biotechnology (2008) Vol. 26, No. 4:443-452
Non Patent Literature 2: K. A. D' Amour et al., Nature Biotechnology (2006) Vol. 24, No. 11:1392-1401
Non Patent Literature 3: W. Jiang, Cell Research (2007) 17:333-344
Non Patent Literature 4: J. H. Shim et al., Diabetologia (2007) 50:1228-1238
Non Patent Literature 5: R. Maehr et al., PNAS (2009), vol. 106, No. 37:15768-15773
Non Patent Literature 6: Kunisada Y et al., Stem Cell Res. (2012) vol. 8, No. 2:274-284
Non Patent Literature 7: Kroon E et al., Nat Biotechnol. (2008) vol. 26, No. 4:443-452
Non Patent Literature 8: Rezania A et al., Diabetes. (2012) vol. 61, No. 8:2016-2029.

### SUMMARY OF INVENTION

The present invention is defined in the claims.

In one aspect, an object of the present application is to provide a method for inducing differentiation of PDX1⁺/NKX6.1⁻ cells into pancreatic bud cells. More specifically, an object of the present application is to provide a method including the step of inducing differentiation of pluripotent stem cells into PDX1⁺/NKX6.1⁻ cells and then, into pancreatic bud cells. Further described herein is a therapeutic agent for treating a pancreatic disease as well as its use in a method for treating a pancreatic disease.

The present inventors have studied diligently in order to achieve the objects described above and, as a result, found for the first time that differentiation of PDX1⁺/NKX6.1⁻ cells into pancreatic bud cells can be efficiently induced by culturing the PDX1⁺/NKX6.1⁻ cells in a medium comprising KGF, EGF, a BMP inhibitor, and an Akt inhibitor. Furthermore, the present inventors have found for the first time that Akt inhibitors have a function to specifically proliferate PDX1⁺ cells. The present invention has been completed based on such findings.

### EFFECTS OF THE INVENTION

According to the present invention, pancreatic bud cells are efficiently induced from PDX1⁺/NKX6.1⁻ cells, as defined in the claims.

### BRIEF DESCRIPTION OF DRAWINGS

[Figure 1] Figure 1 illustrates a protocol for producing pancreatic bud cells from pluripotent stem cells.
[Figure 2] Figure 2 illustrates the number of cells obtained by culturing the cells in the presence of the indicated concentrations of AT7867 in the medium in step 3 (Stage 3).
[Figure 3] Figure 3 illustrates the ratio of PDX1⁺ cells (left panel), PDX1⁺/Ki67⁺ cells (center panel), and PDX1⁻/Ki67⁺ cells (center panel) in the culture on the indicated days in step 3 (Stage 3).
[Figure 4A] Figure 4A illustrates immunostaining images with an anti-NKX6.1 antibody (upper panels) and nuclear staining images (lower panels) after step 3 (Stage 3). White color represents the stained area.
[Figure 4B] Figure 4B illustrates the ratio of NKX6.1⁺ cells when the cells were cultured in the presence of the indicated concentrations of AT7867.
[Figure 5] Figure 5A illustrates a result of flow cytometry of a cell population stained with an anti-PDX1 antibody and an anti-NKX6.1 antibody after step 3 (Stage 3). Figure 5B illustrates the ratio between PDX1⁺/NKX6.1⁻cells and PDX1⁺/NKX6.1⁺ cells obtained from the result of the flow cytometry.
[Figure 6] Figure 6 illustrates the expression of PDX1, PTF1A, and NKX6.1 determined by quantitative PCR every day during the period from the start to the end (Day 0-4) of the step 3 (Stage 3). The solid line represents the change of gene expression when AT7867 was added and the dashed line represents the change when DMSO was added (control group).

### DESCRIPTION OF EMBODIMENTS

The present application provides, in one aspect, a method for generating pancreatic bud cells, comprising the step of culturing PDX1⁺/NKX6.1⁻ cells in a medium containing KGF, EGF a BMP inhibitor and an Akt inhibitor.

Pancreatic bud cells refer to cells which are capable of differentiating into cells which constitute the pancreas such as endocrine cells, pancreatic duct cells and exocrine cells. Examples of pancreatic bud cells include cells which express at least PDX1 and NKX6.1. The pancreatic bud cells may also express genetic markers such as SOX9 and GATA4.

The pancreatic bud cells generated according to this aspect may be provided as a cell population which also contains cells other than pancreatic bud cells, or as a purified cell population. Preferably, the cell population contains pancreatic bud cells by 30% or more, 40% or more, 50% or more, 60% or more, 70% or more, or 80% or more.

Upon producing pancreatic bud cells from PDX1⁺/NKX6.1⁻ cells, the PDX1⁺/NKX6.1⁻ cells may be cultured under adherent culture conditions in a medium comprising KGF, EGF, a BMP inhibitor, and an Akt inhibitor.

In the specification and claims of the present application, adherent cultures refer to any of culturing methods wherein culture dish used for the culture is suitably coated. Examples of coating materials include Matrigel (BD Biosciences), Synthemax (Corning), gelatins, extracellular proteins, for example, collagen, laminin such as laminin-111, -411 or -511, heparan sulfate proteoglycan and entactin, fragments of the extracellular protein and combinations thereof.

Upon producing pancreatic bud cells from PDX1⁺/NKX6.1⁻ cells, adherent culture is preferred because it is easy to handle. Suspension culture in which the cells aggregate in the medium may also be employed.

Suspension cultures refer to cultures in which cells are cultured in a state where the cells do not adhere to the culture dish. Suspension cultures may be performed by using, but not particularly limited to, a culture dish which has not been treated to improve cellular adhesiveness (for example, extracellular matrix coatings), or a culture dish which has been treated to suppress cellular adhesion (for example, poly-hydroxyethyl methacrylate (poly-HEMA) coating or a polymer of 2-methacryloyloxyethylphosphorylcholine (Lipidure) coating).

The medium used for culturing PDX1⁺/NKX6.1⁻ cells to generate pancreatic bud cells can be prepared by adding KGF, EGF, a BMP inhibitor and an Akt inhibitor appropriately to a basal medium for animal cell culture. Examples of basal media include MEM Zinc Option, IMEM Zinc Option, IMDM, Medium 199, Eagle's Minimum Essential Medium (EMEM), α-MEM, Dulbecco's modified Eagle's Medium (DMEM), Ham's F12 Medium, RPMI 1640 Medium, Fischer's Medium, and mixtures of these media. The basal medium may be supplemented with serum, for example, fetal bovine serum (FBS). Alternatively, be a serum-free medium may be used. As required, the basal medium may contain, for example, one or more alternatives to sera such as albumin, transferrin, KnockOut Serum Replacement (KSR, an alternative to serum used for culturing ES cells) (Invitrogen), N2 Supplement (Invitrogen), B27 Supplement (Invitrogen), a fatty acid, insulin, a collagen precursor, a trace element, 2-mercaptoethanol and 3'-Thioglycerol, and the basal medium may contain one or more substances such as a lipid, an amino acid, L-glutamine, GlutaMAX (Invitrogen), a nonessential amino acid (NEAA), a vitamin, a growth factor, an antibiotic, an antioxidant, pyruvic acid, a buffer agent, an inorganic salt, and equivalents thereof. In one embodiment, the basal medium is IMEM Zinc Option containing B-27 Supplement.

The Akt inhibitor is not limited as long as it is a substance that suppresses the activity of Akt. Examples thereof include Akt Inhibitors (Akt Inhibitor, Akt Inhibitor II to XIII) available from Merck Millipore, MK-2206, Perifosine (KRX-0401), GSK690693, AT7867, Ipatasertib (GDC-0068), AZD5363, SC79, Afuresertib (GSK2110183), AT13148, TIC10, Triciribine, CCT128930, PHT-427, Palomid 529, PF-04691502, Honokiol, Miltefosine, Deguelin, A-674563, sc-221226, anti-Akt antibodies, and dominant negative mutants of Akt. These substances are either commercially available or those that can be produced based on known techniques as appropriate.

When AT7867 is used as an Akt inhibitor, its concentration in the medium may be 0.01 µM to 4 µM, preferably 0.1 µM to 1 µM, and more preferably 0.5 µM.

KGF is a protein called Keratinocyte Growth Factor and is sometimes also referred to as FGF-7. KGF is commercially available from, for example, R&D systems, Inc. The concentration of KGF may be from 1 ng/ml to 1 µg/ml, preferably from 5 ng/ml to 500 ng/ml, and more preferably from 10 ng/ml to 200 ng/ml.

EGF is a protein called epidermal growth factor. EGF is commercially available from, for example, R&D systems, Inc. The concentration of EGF may be from 1 ng/ml to 1 µg/ml, preferably from 5 ng/ml to 500 ng/ml, and more preferably from 10 ng/ml to 100 ng/ml.

Examples of the BMP inhibitors include protein inhibitors such as Chordin, Noggin and Follistatin; Dorsomorphin or 6-[4-(2-piperidin-1-yl-ethoxy)phenyl]-3-pyridin-4-yl-pyrazolo[1,5-a]pyrimidine and a derivative thereof (P. B. Yu et al. (2007), Circulation, 116: II 60, P. B. Yu et al. (2008), Nat. Chem. Biol., 4:33-41, J. Hao et al. (2008), PLoS ONE, 3 (8): e2904), and LDN-193189 or4-(6-(4-(piperazin-1-yl)phenyl)pyrazolo[1,5-a]pyrimidin-3-yl)quinoline. Preferable BMP inhibitor is Noggin. Noggin is commercially available, for example, from Peprotech.

When LDN-193189 is used as a BMP inhibitor, the concentration of LDN-193189 may be 0.01 µM to 5 µM, preferably 0.1 µM to 1 µM, and more preferably 0.2 µM.

The medium used in the step of culturing the PDX1⁺/NKX6.1⁻ cells to provide the pancreatic bud cells as described herein may further comprise at least one compound selected from the group consisting of ROCK inhibitors, non-muscle myosin II inhibitors, and TGF β inhibitors.

The ROCK inhibitor is not particularly limited as long as it can suppress the function of Rho-kinase (ROCK), and examples of ROCK inhibitors include Y-27632 (for example, see Ishizaki et al., Mol. Pharmacol. 57, 976-983 (2000); Narumiya et al., Methods Enzymol. 325, 273-284 (2000)), Fasudil/HA1077 (Uenata et al., Nature 389: 990-994 (1997)), SR3677 (Feng Y et al., J Med Chem. 51: 6642-6645 (2008)), GSK269962 (Stavenger RA et al., J Med Chem. 50: 2-5 (2007) or WO 2005/037197), H-1152 (Sasaki et al., Pharmacol. Ther. 93: 225-232 (2002)), Wf-536 (Nakajima et al., Cancer Chemother Pharmacol. 52 (4): 319-324 (2003)) and derivatives thereof, as well as an antisense nucleic acid against ROCK, an RNA interference-inducing nucleic acid (for example, an siRNA) against ROCK, a dominant negative mutant of ROCK, and expression vectors for them. In addition, as the ROCK inhibitor, other known low molecular weight compounds may be used (U.S. patent publications Nos. 2005/0209261, 2005/0192304, 2004/0014755, 2004/0002508, 2004/0002507, 2003/0125344 and 2003/0087919, as well as International Publications Nos. WO 2003/062227, WO 2003/059913, WO 2003/062225, WO 2002/076976, and WO 2004/039796). In the present invention, one or more kinds of ROCK inhibitors may be used. Examples of the ROCK inhibitors which are preferably used in this step include Y-27632, Fasudil/HA1077, SR3677, GSK269962 and H-1152.

When Y-27632 is used as a ROCK inhibitor, its concentration in the medium may be 0.1 µM to 100 µM, preferably 1 µM to 500 µM, and further preferably 10 µM to 200 µM.

When Fasudil/HA1077 is used as a ROCK inhibitor, its concentration in the medium may be 1 µM to 100 µM and preferably 10 µM to 100 µM.

When SR3677 is used as a ROCK inhibitor, its concentration in the medium may be 0.1 µM to 50 µM and preferably 0.5 µM to 50 µM.

When GSK269962 is used as a ROCK inhibitor, its concentration in the medium may be 0.001 µM to 100 µM, preferably 0.005 µM to 50 µM, and further preferably from 0.05 µM to 10 µM.

When H-1152 is used as a ROCK inhibitor, its concentration in the medium may be 5 µM to 100 µM and preferably 10 µM to 50 µM.

The nonmuscle myosin II inhibitor may be an ATPase inhibitor that inhibits the ATPase activity of the heavy chain subunit of nonmuscle myosin IIA, nonmuscle myosin IIB which is one of heavy chain isoforms of nonmuscle myosin II or a myosin light chain kinase inhibitor. Examples of the nonmuscle myosin II inhibitors may include blebbistatin (Blebbistatin) A3, Calphostin C, Goe6976, Goe7874, Fasudil/HA1077, Hypericin, K-252a, KT5823, ML-7, ML-9, Piceatannol, Staurosporine, W-5, W-7, W-12, W-13 and Wortmannin. Preferred nonmuscle myosin II inhibitors are blebbistatin and Fasudil/HA1077.

When Blebbistatin is used as a non-muscle myosin II inhibitor in the present invention, its concentration in the medium may be 1 µM to 200 µM and preferably 10 µM to 100 µM.

The TGFβ inhibitor is a substance which inhibits the signal transduction starts from the binding of TGFβ to its receptor and leads to SMAD. The TGFβ inhibitor is not particularly limited as long as it inhibits the binding of TGFβ to the receptor, an ALK family protein, or inhibits phosphorylation of SMAD caused by the ALK family protein. Examples of TGFβ inhibitors include Lefty-1 (NCBI Accession Nos: NM_010094 (mouse), and NM_020997(human)), SB431542 and SB202190 (R. K. Lindemann et al., Mol. Cancer, 2003, 2: 20), SB505124 (GlaxoSmithKline), NPC30345, SD093, SD908, SD208 (Scios), LY2109761, LY364947, LY580276 (Lilly Research Laboratories), A-83-01 (WO 2009/146408), ALK5 inhibitor II (2-[3-[6-methylpyridin-2-yl]-1H-pyrazol-4-yl]-1,5-naphthyridine), TGFβRI kinase inhibitor VIII (6-[2-tert-butyl-5-[6-methyl-pyridin-2-yl]-1H-imidazol-4-yl]-quinoxaline) and derivatives thereof. Preferably, the TGFβ inhibitor may be ALK5 inhibitor II.

When ALK5 inhibitor II is used as a TGF β inhibitor, its concentration in the medium may be 0.01 µM to 100 µM, preferably 0.1 µM to 50 µM, and more preferably from 1 µM to 20 µM.

The number of days of the step of culturing the PDX1⁺/NKX6.1⁻ cells to the pancreatic bud cell has no upper limit since the production efficiency of pancreatic bud cells is not particularly affected by culturing the cell for a long period of time, but it may be, for example, 2 days or more, 3 days or more, 4 days or more, 5 days or more, 6 days or more, 7 days or more, 8 days or more, 9 days or more, 10 days or more, 11 days or more, 12 days or more, 13 days or more, or 14 days or more. It is preferably equal to or longer than 3 days and equal to or shorter than 20 days, more preferably equal to or longer than 4 days, and equal to or shorter than 20 days, and, for example, 4 days.

In the step of culturing PDX1⁺/NKX6.1⁻ cells to generate pancreatic bud cells, the cells may be cultured at a temperature from about 30°C to about 40°C, and preferably about 37°C. In this step, the cells are cultured under a CO₂-containing air atmosphere, and the concentration of CO₂ is preferably from about 2% to about 5%.

PDX1⁺/NKX6.1⁻ cells are not particularly limited as long as the cells express PDX1 but do not express NKX6.1. The phrase "cells express PDX1" means that PDX1 gene or PDX1 gene product can be detected in the cells by a known method, and the phrase "cells do not express NKX6.1" means that NKX6.1 gene or NKX6.1 gene product cannot be detected by any known method. Examples of the known detection method include immunostaining.

As another aspect of the present application, a method for proliferating PDX1-positive cells, comprising the step of culturing PDX1-positive cells in a medium comprising KGF, EGF, a BMP inhibitor, and an Akt inhibitor is provided. The PDX1-positive cell means a cell that express PDX1. Expression of marker genes other than PDX1, such as NKX6.1 on the cell is not particularly limited and the PDX1 positive cells used in this step are preferably NKX6.1 negative cells. Proliferating PDX1-positive cells means that cells positive for at least PDX1 increase by cell division and the proliferation may be confirmed, for example, by confirming that the number of cells stained with Ki67 antibody increases. The PDX1-positive cells can be proliferated under conditions same as those used in the step of culturing the PDX1⁺/NKX6.1⁻cells to provide the pancreatic bud cell described above.

PDX1⁺/NKX6.1⁻ cells may have been isolated from the living body or may be induced from other types of cells such as pluripotent stem cells by a known method. In a certain embodiment, PDX1⁺/NKX6.1⁻ cells may be those generated from pluripotent stem cells by a method comprising the following steps:
(Step 1) culturing the pluripotent stem cells in a medium containing an activin; and
(Step 2) culturing the cells obtained in Step 1 in a medium containing KGF.

In one embodiment, a method for generating pancreatic bud cells from pluripotent stem cells including the step of inducing pancreatic bud cells from PDX1⁺/NKX6.1⁻ cells as Step 3 of the above-described method is provided:
(Step 1) culturing the pluripotent stem cells in a medium containing an activin;
(Step 2) culturing the cells obtained in Step 1 in a medium containing KGF; and
(Step 3) dissociating the cells (PDX1⁺/NKX6.1⁻ cells) obtained in Step 2 into single cells and culturing the cells in a medium containing KGF, EGF, a BMP inhibitor and an Akt inhibitor.

The medium used for culturing the pluripotent stem cells in a medium containing an activin (Step 1) according to these embodiments can be prepared by adding an activin to a basal medium for animal cells. Examples of the basal media include MEM Zinc Option, IMEM Zinc Option, IMDM, Medium 199, Eagle's Minimum Essential Medium (EMEM), α-MEM , Dulbecco's modified Eagle's Medium (DMEM), Ham's F12 Medium, RPMI 1640 Medium, Fischer's Medium, and mixtures of these media. The basal medium may be supplemented with serum, for example, fetal bovine serum (FBS), or the basal medium may be a serum-free medium. As required, the basal medium may contain, for example, one or more alternatives to sera such as albumin, transferrin, KnockOut Serum Replacement (KSR) (an alternative to serum used for culturing ES cells) (Invitrogen), N2 Supplement (Invitrogen), B-27 Supplement (Invitrogen), a fatty acid, insulin, a collagen precursor, a trace element, 2-mercaptoethanol and 3'-Thioglycerol, and the basal medium may contain one or more substances such as a lipid, an amino acid, L-glutamine, GlutaMAX (Invitrogen), a nonessential amino acid (NEAA), a vitamin, a growth factor, an antibiotic, an antioxidant, pyruvic acid, a buffer agent, an inorganic salt, and equivalents thereof. In one embodiment, the basal medium is RPMI 1640 medium containing B-27 Supplement.

During Step 1 in this embodiment, single cell suspension of pluripotent stem cells may be prepared by substantially dissociating or decomposing the cellular aggregates by a conventional procedure and cultured. Alternatively, cellular aggregates of pluripotent stem cells in which cells adhere each other may be cultured. Preferably, suspension culture of single cells may be employed. Examples of the procedures for dissociating cells include mechanical dissociation, chemical dissociation using a dissociation solution such as a solution containing both protease and collagenase activities such as a solution containing trypsin and collagenase, Accutase^{™}, Accumax^{™} (Innovative Cell Technologies, Inc.)) or a solution having only collagenase activity. Pluripotent stem cells may be cultured in adherent cultures by using a coated culture dish.

A ROCK inhibitor may be added to the basal medium for the purpose of suppressing apoptosis in separating the pluripotent stem cells into single cells. The ROCK inhibitor to be used may be those described above and preferably Y-27632. When a ROCK inhibitor is added for this purpose, the period for culturing the cells in the presence of the ROCK inhibitor is not particularly limited and may be one or more days or 2 or more days, and preferably one day.

The adherent culture in stage 1 of this embodiment may be performed under the same conditions as those of the method described above.

As an activin, any activins including activin A, activin B, activin C, activin D and activin AB may be used, and activin A is preferable. In addition, as an activin, any one of activins derived from mammals such as human and mouse may be used. As the activin used for the present invention, an activin derived from the same animal as the animal from which pluripotent stem cells used for differentiation are derived is preferably used, and for example, when pluripotent stem cells derived from human are used as starting materials, an activin derived from human is preferably used. These activins are commercially available.

When activin is used, its concentration in the medium may be 0.1 to 200 ng/ml, preferably 5 to 150 ng/ml, and more preferably 10 to 100 ng/ml.

In step 1, the cells may be cultured in a medium comprising an activin and a GSK3 inhibitor and then in a medium comprising activin but no GSK3 inhibitor.

A GSK3 inhibitor is defined as a substance which inhibits the kinase activity of GSK-3β protein such as an ability to phosphorylate β-catenin, and many GSK3 inhibitors are known. Examples of the GSK3 inhibitors include BIO (also called GSK-3β inhibitor IX; 6-bromoindirubin3'-oxime) which is a derivative of indirubin, SB216763 (3-(2,4-dichlorophenyl)-4-(1-methyl-1H-indol-3-yl)-1H-pyrrole-2,5-dione) and SB415286 (3-[(3-chloro-4-hydroxyphenyl)amino]-4-(2-nitrophenyl)-1H-pyrrole-2,5-dione) which are derivatives of maleimide, GSK-3β inhibitor VII (4-dibromoacetophenone) which is a phenyl α bromomethylketone compound, L803-mts (also called, GSK-3β peptide inhibitor; Myr-N-GKEAPPAPPQSpP-NH2) which is a cell-penetrating phosphorylated peptide and CHIR99021 (6-[2-[4-(2,4-Dichlorophenyl)-5-(4-methyl-1H-imidazol-2-yl)pyrimidin-2-ylamino]ethylamino]pyridine-3-carbonitrile) which has a high selectivity. These compounds are commercially available, for example, from Calbiochem and Biomol. These compounds may be obtained from other suppliers or may be prepared by the user himself. The GSK-3β inhibitor used for the present invention may preferably be CHIR99021.

When CHIR99021 is used as a GSK-3β inhibotor, its concentration in the medium may be usually 0.01 µM to 100 µM, preferably 0.1 µM to 10 µM, and more preferably from 1 µM to 5 µM. The concentration may be changed as appropriate and, for example, it may be changed from 3 µM to 1 µM.

When step 1 include the step of culturing the cells in the presence of an activin and a GSK3 inhibitor, it is desirable to start the step 1 by adding them to the pluripotent stem cell culture. The period for culturing the cells in the presence of the GSK inhibitor is not particularly limited and may be one or more days, 2 or more days, or 3 or more days, and preferably 1 to 3 days. After that, the cells are cultured in a medium comprising activin but no GSK3 inhibitor.

The number of days of step 1 may be, for example, 3 days or more, 4 days or more, 5 days or more, 6 days or more, or 7 days or more. There is no upper limit since the production efficiency of pancreatic bud cells is not affected by culturing the cell for a long period of time. Preferably it is 10 days or less and more preferably 8 days or less from the viewpoint of efficiency. More preferably, the number of days of step 1 in total may be 4 days.

In one embodiment, pluripotent cells are cultured for 1 day in a medium containing a GSK3 inhibitor and a ROCK inhibitor in addition to activin A, then cultured for 2 days in a medium comprising activin A and a GSK3 inhibitor but no ROCK inhibitor, and then cultured for further 1 day in a medium comprising activin A, but no GSK3 inhibitor or no ROCK inhibitor.

In step 1, the culturing temperature is, but not limited to, about 30-40°C, preferably about 37°C and the culturing is performed in the atmosphere of CO₂-containing air with preferred CO₂ concentrations being about 2-5%.

In step 2, the medium used for culturing the cells obtained in step 1 may be prepared by adding KGF to a basal medium for animal cells. Examples of the basal media may include MEM Zinc Option, IMEM Zinc Option, IMDM, Medium 199, Eagle's Minimum Essential Medium (EMEM), α-MEM, Dulbecco's modified Eagle's Medium (DMEM), Ham's F12 Medium, RPMI 1640 Medium, Fischer's Medium, and mixtures of these media. The basal medium may contain serum (for example, fetal bovine serum (FBS)) or the basal medium may be a serum-free medium. As required, the basal medium may contain, for example, one or more alternatives to sera such as albumin, transferrin, KnockOut Serum Replacement (KSR) (an alternative to serum used for culturing ES cells) (Invitrogen), N2 Supplement (Invitrogen), B-27 Supplement (Invitrogen), a fatty acid, insulin, a collagen precursor, a trace element, 2-mercaptoethanol and 3'-Thioglycerol, and the basal medium may contain one or more substances such as a lipid, an amino acid, L-glutamine, GlutaMAX (Invitrogen), a nonessential amino acid (NEAA), a vitamin, a growth factor, an antibiotic, an antioxidant, pyruvic acid, a buffer agent, an inorganic salt, and equivalents thereof. In one embodiment, the basal medium is IMEM Zinc Option containing B-27 Supplement.

KGF used in step 2 may be the same as the above-described KGF. The concentration of KGF in step 2 may preferably be lower than the above-described concentration, and for example, the concentration of KGF in step 2 may be from 1 ng/ml to 500 ng/ml, and preferably from 10 ng/ml to 100 ng/ml.

Step 2 may further comprise the step of culturing the cells in a medium comprising a BMP inhibitor, a retinoic acid derivative, and a hedgehog pathway inhibitor, in addition to KGF.

The BMP inhibitor to be used in step 2 may be used under conditions as described above.

The retinoic acid derivative to be used in step 2 means retinoic acids artificially modified and maintaining the function of natural retinoic acid and examples thereof include 4-[[(5,6,7,8-tetrahydro-5,5,8,8-tetramethyl-2-naphthalenyl)carbonyl]amino]-Benzoic acid (AM580) (Tamura K, et al., Cell Differ. Dev. 32:17-26 (1990)), 4-[(1E)-2-(5,6,7,8-tetrahydro-5,5,8,8-tetramethyl-2-naphthalenyl)-1-propen-1-yl]-Benzoic acid (TTNPB) (Strickland S, et al., Cancer Res. 43: 5268-5272 (1983)), retinol palmitate, retinol, retinal, 3-dehydroretinoic acid, 3-dehydroretinol, 3-dehydroretinal, or the compounds described in Abe, E., et al., Proc. Natl. Acad. Sci. (USA) 78:4990-4994 (1981); Schwartz, E. L. et al., Proc. Am. Assoc. Cancer Res. 24:18 (1983); and Tanenaga, K. et al., Cancer Res. 40:914-919 (1980). Preferred examples of the retinoic acid derivatives to be used in this step include TTNPB. A person skilled in the art can select the concentration of the retinoic acid derivative to be used in this step depending on the retinoic acid derivative to be used as appropriate, but, for example, when TTNPB is used as the retinoic acid derivative, its concentration in the medium may be 1 nM to 100 nM, preferably 5 nM to 50 nM, and further preferably 5 nM to 10 nM.

The hedgehog pathway inhibitor to be used in step 2 means a compound that inhibits the activity of signal, for example, Smoothened, generated by binding of any of Sonic Hedgehog, Indian Hedgehog, and Desert Hedgehog to the membrane receptor Patched and is not particularly limited as long as it inhibits the signal generated by binding of hedgehog to the receptor, but examples thereof include cyclopamine, Jervine, 3-Keto-N-(aminoethyl-aminocaproyl-dihydro-cinnamoyl) (KAAD)-cyclopamine, CUR-61414, SANT-1, SANT-2, SANT-3, SANT-4, IPI-926, IPI-269609, GDC-0449, and NVP-LDE-225. Preferably, it is KAAD-cyclopamine. Person skilled in the art can select the concentration of the hedgehog pathway inhibitor to use in step 2 depending on hedgehog pathway inhibitor to use as appropriate, but, for example, the concentration is from 0.1 nM to 1 µM, preferably from 1 nM to 500 nM when KAAD-cyclopamine is used as hedgehog pathway inhibitor.

When performing adherent culture in a medium comprising KGF, a BMP inhibitor, a retinoic acid derivative, and a hedgehog pathway inhibitor in step 2, cultured cells may be substantially separated (or dissociated) into single cells by any method in the middle of the culture and the single cell suspension may be cultured under the same conditions again. In this operation, a ROCK inhibitor may be added to the basal medium for the purpose of suppressing apoptosis in separating the cells into single cells. The ROCK inhibitor to be used may be those described above and preferably Y-27632. When a ROCK inhibitor is added for this purpose, the duration of treatment is not particularly limited and examples thereof include one or more days or 2 or more days, but preferably one day.

The number of days of step 2 may be for example, 4 days or more, 5 days or more, 6 days or more, 7 days or more, or 8 days or more. There is no upper limit since the production efficiency of pancreatic bud cells are not affected by culturing the cells for a longer period of time. It is preferably 10 days or less or 9 days or less from the viewpoint of efficiency. More preferably, the number of days is 7 days. When culturing the cells in a medium comprising KGF and then culturing in a medium comprising KGF, a BMP inhibitor, a retinoic acid derivative, and a hedgehog pathway inhibitor, the number of days of culture in the medium comprising KGF may be 3 days or more or 4 days or more and more preferably 4 days.

In one embodiment, the culture medium of the cells obtained by the adherent culture in step 1 is changed to a medium comprising KGF and to start step 2, i.e. the cells are cultured in the medium comprising KGF for 4 days. Then, the medium is again changed to a medium comprising a BMP inhibitor, a retinoic acid derivative, and a hedgehog inhibitor in addition to KGF and the cells are cultured for 2 days. After that, the cells are separated from the inner surface of the culture container and dissociated into single cell suspension, and the cells are further cultured for 1 day in a medium comprising a ROCK inhibitor.

In step 2, the culturing temperature may be, but not limited to, about 30-40°C, preferably about 37°C and the culturing is carried out in the atmosphere of CO₂-containing air with preferred CO₂ concentrations being about 2-5%. Cells obtained at the end of step 2 are PDX1⁺/NKX6.1⁻ cells. The cells obtained at the end of step 2 may be provided as a cell population containing cells other than PDX1⁺/NKX6.1⁻ cells or may be a purified population. Preferably, the cells are provided as a cell population containing 50% or more, 55% or more, 60% or more, 65% or more, or 70% or more PDX1⁺/NKX6.1⁻ cells.

In the specification and claims of the present application, pluripotent stem cells refer to stem cells which have pluripotency, that is the ability of cells to differentiate into all type of the cells in the living body, as well as proliferative capacity. Reference examples of the pluripotent stem cells include embryonic stem (ES) cells (J. A. Thomson et al. (1998), Science 282: 1145-1147; J. A. Thomson et al. (1995), Proc. Natl. Acad. Sci. USA, 92: 7844-7848; J. A. Thomson et al. (1996), Biol. Reprod., 55: 254-259; J.A. Thomson and V.S. Marshall (1998), Curr. Top. Dev. Biol., 38: 133-165), nuclear transfer embryonic stem (ntES) cells that can be obtained by nuclear transplantation into the ES cells(T. Wakayama et al. (2001), Science, 292: 740-743; S. Wakayama et al. (2005), Biol. Reprod., 72: 932-936; J. Byrne et al. (2007), Nature, 450: 497-502), germline stem cells ("GS cells") (M. Kanatsu-Shinohara et al. (2003) Biol. Reprod., 69: 612-616; K. Shinohara et al. (2004), Cell, 119: 1001-1012), embryonic germ cells ("EG cells") (Y. Matsui et al. (1992), Cell, 70: 841-847; J. L. Resnick et al. (1992), Nature, 359: 550-551), induced pluripotent stem (iPS) cells (K. Takahashi and S. Yamanaka (2006) Cell, 126: 663-676; K. Takahashi et al. (2007), Cell, 131:861-872; J. Yu et al. (2007), Science, 318: 1917-1920; Nakagawa, M. et al., Nat. Biotechnol. 26: 101-106 (2008); WO 2007/069666), pluripotent cells derived from cultured fibroblasts and bone marrow stem cells (Multi-lineage differentiating Stress Enduring cells, Muse cells) (WO 2011/007900). More preferably, the pluripotent stem cells are human pluripotent stem cells.

The iPS cells are particularly preferable as pluripotent stem cells for generating pancreatic bud cells used for implantation. For therapeutic use, it is desirable that the iPS cells are those induced from somatic cells of an individual who has the same or substantially the same HLA genotypes as those of the individual who receives implantation of the cells, from the viewpoint that the cells do not cause rejection reaction. In this regard, the phrase "substantially the same HLA" means HLAs between the donner and the recipient match to a degree that the immunoreaction against the implanted cells can be suppressed by an immunosuppressant. For example, substantially the same HLA may cover three genes match, i.e. HLA-A, HLA-B, and HLA-DR match or four genes match, i.e. HLA-A, HLA-B, HLA-DR and HLA-C mach.

Pancreatic bud cells obtained through the above-described procedures may be used as a medicament, in particular, for cellular therapy, as described herein. The cells may be, before being administered, irradiated with radiation or treated with a compound which suppresses proliferation of the cells such as mitomycin C.

The pancreatic bud cells may be suspended in physiological saline or the like and the suspension may be administered directly to the pancreas, the mesenterium, the spleen, the liver or the kidney, in particular into the kidney subcapsule of the patient. Alternatively, the cells may be encapsulated with polyvinyl alcohol (PVA) (Qi Z et al., Cell Transplant. 21: 525-534 (2012)) or alginic acid (Dufrane D, et al., Transplantation 90: 1054-1062 (2010)) and the capsule may be administered. The cells may be administered in combination with a scaffolding material such as polyethylene glycol, gelatin, or collagen. The number of the cells administered may be appropriately determined depending on the body size, and may be, for example, from 1 × 10⁸ to 1 × 10¹⁰ cells/body, is preferably from 5 × 10⁸ to 1 × 10¹⁰ cells/body, and more preferably from 1 × 10⁹ to 1 × 10¹⁰ cells/body.

The pancreatic bud cells may be used for treating a pancreatic disease, as described herein. Examples of the pancreatic diseases include acute pancreatitis, chronic pancreatitis, diabetes, pancreatic cancer and islet of Langerhans tumor. The pancreatic bud cells according to the present invention are induced to be insulin-producing cells which secrete insulin in response to glucose level in the body and are effective for treating diabetes. In particular, the medicament containing the pancreatic bud cells are effective for treating type 1 diabetes mellitus in which insulin-producing cells die.

The origin of the cells described in the specification are not particularly limited and may include human and nonhuman animals, for example, mice, rats, cattle, horses, pigs, sheep, monkeys, dogs, cats and birds. Human cells are preferably used.

The present invention is described in more detail referring to following Examples. The present invention, however, is not limited by those Examples in any way. Examples

### IPS cell line

An iPS cell line (585A1) derived from a peripheral blood mononuclear cell (Okita K, et al, Stem Cells. 2013 31:458-466) has been available from Center for iPS Cell Research and Application, Kyoto University. Cells of iPS cell line 585A1 obtained from Kyoto University were differentiated into pancreatic bud cells by the following steps.

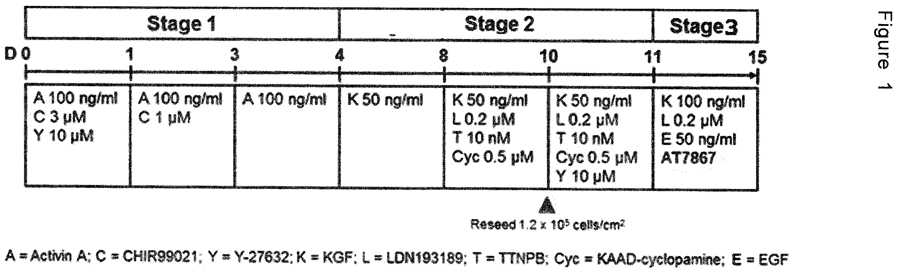

### Stages of inducing differentiation (Figure 1)

### (Stage 1)

Cells of iPS cell line (585A1) were seeded at 2.0 × 10⁵/well in a 24 well plate coated with Matrigel and cultured for 1 day in RPMI1640 medium (Nacalai Tesque, Inc.) containing 2% B27 (Life Technologies) supplemented with 100 ng/ml activin A (R&D systems, Inc.), 3 µM CHIR99021 (Axon Medchem), and 10 µM Y-27632 (WaKo). The medium was changed to RPMI1640 medium containing 100 ng/ml activin A, 1 µM CHIR99021, and 2% B27 (Life Technologies) and the cells were cultured for 2 days. Furthermore, the medium was changed to RPMI1640 medium containing 100 ng/ml activin A and 2% B27 (Life Technologies) and the cells were cultured for 1 day.

### (Stage 2-1)

The medium in the cell culture obtained in stage 1 was changed to Improved MEM Zinc Option medium (Invitrogen) supplemented with 50 ng/ml KGF (R&D systems, Inc.) and 1% B-27 (Life Technologies) and the cells were cultured for 4 days.

### (Stage 2-2)

The medium in the cell culture obtained in Stage 2-1 was changed to Improved MEM Zinc Option medium supplemented with 50 ng/ml KGF, 0.2µM LDN193189 (Axon Medchem), 10 nM TTNPB (Santa Cruz Biotechnology), 0.5 µM 3-Keto-N-aminoethyl-N'-aminocaproyldihydrocinnamoyl Cyclopamine (KAAD-cyclopamine or K-CYC) (Toronto Research Chemicals) and 1% B-27 and the cells were cultured for 2 days.

### (Stage 2-3)

Subsequently, cells in the culture were dissociated into single cells with Trypsin and seeded at a density of 1.2 × 10⁵/cm² in a 24 well plate coated with Matrigel. Improved MEM Zinc Option medium supplemented with 50 ng/ml KGF, 0.2 µM LDN193189, 10 nM TTNPB, 0.5 µM KAAD-cyclopamine, 10 µM Y-27632, and 1% B-27 was added and the cells were cultured for 1 day.

The cells obtained at the end of Stage 2 were treated with BD Cytofix/Cytoperm(TM) Kit, stained with an anti-PDXl antibody (R&D systems, Inc.) and an anti-NKX6.1 antibody (University of Iowa), and examined with a flow cytometer. The result indicated that approximately 60% of the obtained cells were PDX1⁺/NKX6.1⁻ cells.

### (Stage 3)

The medium in the cell culture obtained in Stage 2-3 was changed to Improved MEM Zinc Option medium (15 × 10⁴/ml) supplemented with 100 ng/ml KGF, 0.2 µM LDN193189, 50 ng/ml EGF (R&D systems, Inc.), indicated concentrations (0.01 µM to 50 µM) of AT7867 (Selleck Chemicals), 1% B-27 (DMSO as negative control) and the cells were further cultured for 4 days.

### Cell analysis

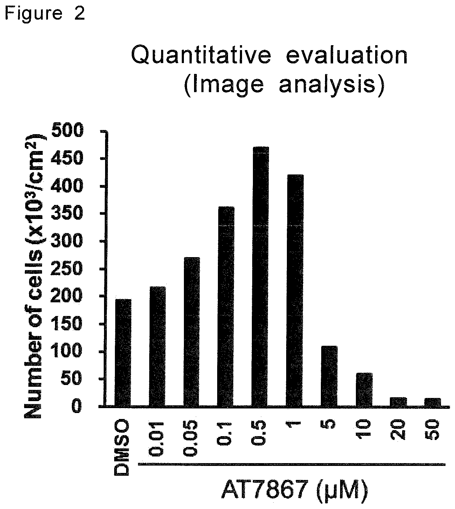

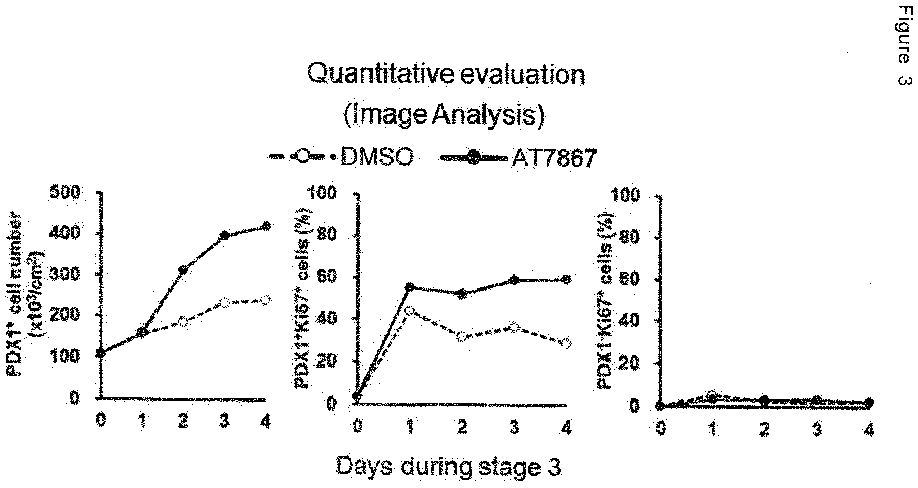

The number of cells obtained in Stage 3 was counted and confirmed that the cell number was increased in a manner dependent on the concentration of AT7867 in the medium in comparison with the cell number of negative control obtained by culturing the cells in the medium containing DMSO(Figure 2). Furthermore, immunostaining of the cells obtained 1 to 4 days after the addition of 1 µM AT7867 in Stage 3 with an anti-PDXl antibody (R&D Systems, Inc.) and an anti-Ki67 antibody (Novocastra) indicated that the proliferated cells were positive for at least PDX1 (Figure 3).

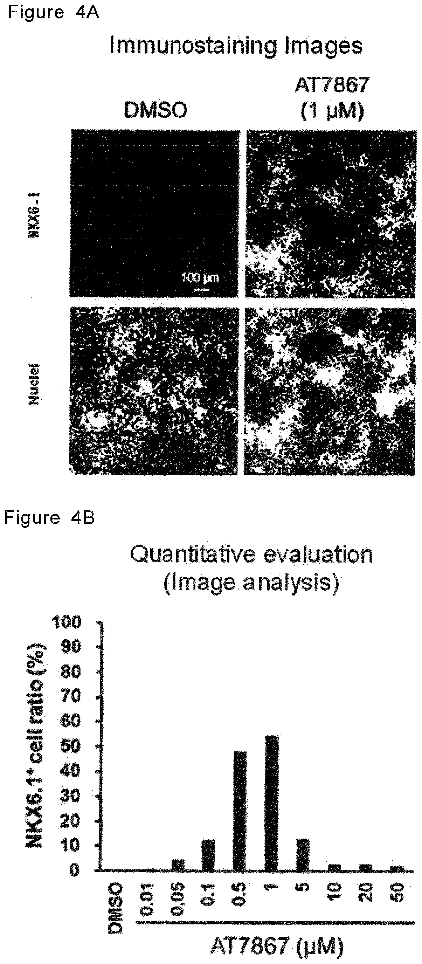

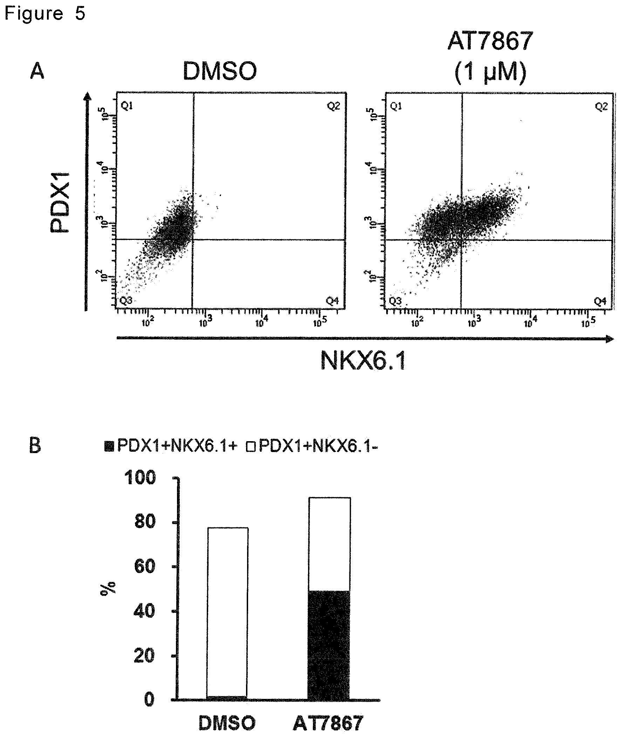

Immunostaining of cells obtained in Stage 3 with an anti-NKX6.1 antibody (DSHB) (Figure 4A) indicated that the cells positive for at least NKX6.1 increased in a manner dependent on the concentration of AT7867 in the medium (Figure 4B). Similarly, double staining with the anti-PDX1 antibody and the anti-NKX6.1 antibody indicated that PDX1⁺/NKX6.1⁺ cells were increased by the addition of AT7867 (Figures 5A and B).

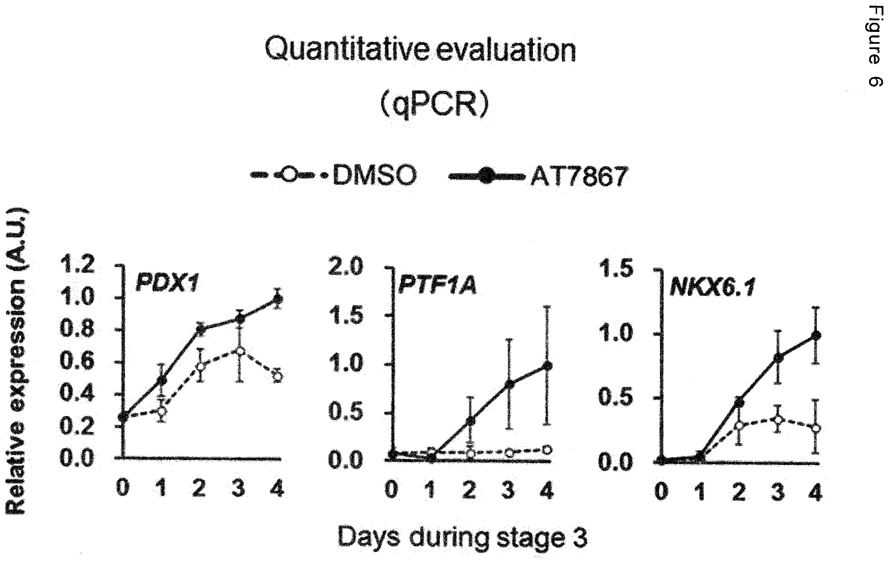

In Stage 3, the cells were cultured in the presence of 1 µM AT7867. Cells were collected every day from Day 0 to Day 4 in Stage 3 and expression of PDX1, PTF1A, and NKX6.1 genes was measured by quantitative PCR. RNAs were extracted from the cells using RNeasy kit (Qiagen) and the reverse transcription reaction was performed with ReverTra Ace qPCR RT Master Mix (Toyobo) and oligo (dT) 20 primer. The quantitative PCR was performed with SYBR Premix Ex Taq II (Takara) using StepOnePlus(TM) Real-Time PCR System (Applied Biosystems). The gene expression was standardized with the expression of GAPDH gene and the relative gene expression was calculated from the relative standard curve (Figure 6). The result indicated that the expression of PDX1 as well as PTF1A and NKX6.1 increased over time after addition of AT7867. PTF1A and NKX6.1 are transcription factors that indicate the differentiation of the cells into pancreatic bud cells

As described in the foregoing, it was suggested that AT7867 induces the differentiation of cells into NKX6.1⁺ cells by specifically increasing PDX1⁺ cells.

## Claims

1. A method for generating pancreatic bud cells, comprising the step of culturing PDX1⁺/NKX6.1⁻ cells in a medium comprising KGF, EGF, a BMP inhibitor and an Akt inhibitor.

2. The method according to claim 1, wherein the cells are cultured under adherent culture conditions.

3. The method according to claim 1 or 2, wherein the Akt inhibitor is AT7867.

4. The method according to any one of claims 1 to 3, further comprising the step of generating the PDX1⁺/NKX6.1⁻ cells from pluripotent stem cells by a method comprising the following steps:
(1) culturing the pluripotent stem cells in a medium containing an activin; and
(2) culturing the cells obtained in step (1) in a medium containing KGF.

5. The method according to claim 4, wherein the step (1) comprises the step of culturing the cells in a medium comprising activin and a GSK3 inhibitor and then in a medium comprising activin but no GSK3 inhibitor.

6. The method according to claim 4 or 5, wherein the step (2) further comprises the step of culturing the cells in a medium comprising KGF, a BMP inhibitor, a retinoic acid derivative, and a hedgehog pathway inhibitor.

7. The method according to claim 6, wherein the step (2) comprises the step of culturing the cells in a medium comprising KGF, a BMP inhibitor, a retinoic acid derivative, and a hedgehog pathway inhibitor, then dissociating the resulting cells, and then culturing the dissociated cells in a medium comprising KGF, a BMP inhibitor, a retinoic acid derivative, and a hedgehog pathway inhibitor under adherent culture conditions.

8. The method according to any one of claims 1 to 7, wherein the BMP inhibitor is LDN193189.

9. The method according to any one of claims 5 to 8, wherein the GSK3 inhibitor is CHIR99021.

10. The method according to any one of claims 6 to 9, wherein the retinoic acid derivative is TTNPB.

11. The method according to any one of claims 6 to 10, wherein the hedgehog pathway inhibitor is KAAD-cyclopamine.

12. The method according to any one of claims 1 to 11, wherein the pancreatic bud cells are PDX1 positive and NKX6.1 positive.

13. The method according to any one of claims 1 to 12, wherein the pancreatic bud cells are human cells.

14. A method for proliferating PDX1-positive cells, comprising the step of culturing PDX1-positive cells in a medium comprising KGF, EGF, a BMP inhibitor, and an Akt inhibitor.

15. The method according to claim 14, wherein the cells are cultured under adherent culture conditions.

## Patentansprüche

1. Verfahren zur Erzeugung von Pankreasknospenzellen (pancreatic bud cells), umfassend den Schritt des Kultivierens von PDX1⁺/NKX6.1⁻ Zellen in einem Medium, das KGF, EGF, einen BMP-Inhibitor und einen Akt-Inhibitor enthält.

2. Verfahren gemäß Anspruch 1, wobei die Zellen unter adhärenten Kulturbedingungen kultiviert werden.

3. Verfahren gemäß Anspruch 1 oder 2, wobei der Akt-Inhibitor AT7867 ist.

4. Verfahren gemäß irgendeinem der Ansprüche 1 bis 3, weitergehend umfassend den Schritt der Erzeugung der PDX1⁺/NKX6.1⁻ Zellen aus pluripotenten Stammzellen durch ein Verfahren, das die folgenden Schritte umfasst:
(1) Kultivieren der pluripotenten Stammzellen in einem Medium, das ein Aktivin enthält; und
(2) Kultivieren der in Schritt (1) erhaltenen Zellen in einem Medium, das KGF enthält.

5. Verfahren gemäß Anspruch 4, wobei der Schritt (1) den Schritt des Kultivierens der Zellen in einem Medium, das Aktivin und einen GSK3-Inhibitor enthält, und dann in einem Medium, das Aktivin, aber keinen GSK3-Inhibitor enthält, umfasst.

6. Verfahren gemäß Anspruch 4 oder 5, wobei der Schritt (2) weitergehend den Schritt des Kultivierens der Zellen in einem Medium umfasst, das KGF, einen BMP-Inhibitor, ein Retinsäurederivat und einen Hedgehog-Signalweg-Inhibitor umfasst.

7. Verfahren gemäß Anspruch 6, wobei der Schritt (2) den Schritt des Kultivierens der Zellen in einem Medium, das KGF, einen BMP-Inhibitor, ein Retinsäurederivat und einen Hedgehog-Signalweg-Inhibitor umfasst, dann das Dissoziieren der resultierenden Zellen und dann das Kultivieren der dissoziierten Zellen unter adhärenten Kulturbedingungen umfasst, in einem Medium, das KGF, einen BMP-Inhibitor, ein Retinsäurederivat und einen Hedgehog-Signalweg-Inhibitor umfasst.

8. Verfahren gemäß irgendeinem der Ansprüche 1 bis 7, wobei der BMP-Inhibitor LDN193189 ist.

9. Verfahren gemäß irgendeinem der Ansprüche 5 bis 8, wobei der GSK3-Inhibitor CHIR99021 ist.

10. Verfahren gemäß irgendeinem der Ansprüche 6 bis 9, wobei das Retinsäurederivat TTNPB ist.

11. Verfahren gemäß irgendeinem der Ansprüche 6 bis 10, wobei der Hedgehog-Signalweg-Inhibitor KAAD-Cyclopamin ist.

12. Verfahren gemäß irgendeinem der Ansprüche 1 bis 11, wobei die Pankreasknospenzellen PDX1-positiv und NKX6.1-positiv sind.

13. Verfahren gemäß irgendeinem der Ansprüche 1 bis 12, wobei die Pankreasknospenzellen menschliche Zellen sind.

14. Verfahren zur Proliferation PDX1-positiver Zellen, umfassend den Schritt des Kultivierens PDX1-positiver Zellen in einem Medium, das KGF, EGF, einen BMP-Inhibitor und einen Akt-Inhibitor enthält.

15. Verfahren gemäß Anspruch 14, wobei die Zellen unter adhärenten Kulturbedingungen kultiviert werden.

## Revendications

1. Procédé de génération de cellules de bourgeon pancréatique, comprenant l'étape de mise en culture de cellules PDX1⁺/NKX6.1⁻ dans un milieu comprenant du KGF, de l'EGF, un inhibiteur de BMP et un inhibiteur d'Akt.

2. Procédé selon la revendication 1, dans lequel les cellules sont mises en culture dans des conditions de culture adhérente.

3. Procédé selon la revendication 1 ou 2, dans lequel l'inhibiteur d'Akt est AT7867.

4. Procédé selon l'une quelconque des revendications 1 à 3, comprenant en outre l'étape de génération des cellules PDX1⁺/NKX6.1⁻ à partir de cellules souches pluripotentes par un procédé comprenant les étapes suivantes :
(1) la mise en culture des cellules souches pluripotentes dans un milieu contenant une activine ; et
(2) la mise en culture des cellules obtenues dans l'étape (1) dans un milieu contenant du KGF.

5. Procédé selon la revendication 4, dans lequel l'étape (1) comprend l'étape de mise en culture des cellules dans un milieu comprenant une activine et un inhibiteur de GSK3 et ensuite dans un milieu comprenant une activine mais sans inhibiteur de GSK3.

6. Procédé selon la revendication 4 ou 5, dans lequel l'étape (2) comprend en outre l'étape de mise en culture des cellules dans un milieu comprenant du KGF, un inhibiteur de BMP, un dérivé d'acide rétinoïque, et un inhibiteur de la voie hedgehog.

7. Procédé selon la revendication 6, dans lequel l'étape (2) comprend l'étape de mise en culture des cellules dans un milieu comprenant du KGF, un inhibiteur de BMP, un dérivé d'acide rétinoïque, et un inhibiteur de la voie hedgehog, puis de dissociation des cellules résultantes, et ensuite de mise en culture des cellules dissociées dans un milieu comprenant du KGF, un inhibiteur de BMP, un dérivé d'acide rétinoïque, et un inhibiteur de la voie hedgehog dans des conditions de culture adhérente.

8. Procédé selon l'une quelconque des revendications 1 à 7, dans lequel l'inhibiteur de BMP est LDN193189.

9. Procédé selon l'une quelconque des revendications 5 à 8, dans lequel l'inhibiteur de GSK3 est CHIR99021.

10. Procédé selon l'une quelconque des revendications 6 à 9, dans lequel le dérivé d'acide rétinoïque est TTNPB.

11. Procédé selon l'une quelconque des revendications 6 à 10, dans lequel l'inhibiteur de la voie hedgehog est la KAAD-cyclopamine.

12. Procédé selon l'une quelconque des revendications 1 à 11, dans lequel les cellules de bourgeon pancréatique sont PDX1-positives et NKX6.1-positives.

13. Procédé selon l'une quelconque des revendications 1 à 12, dans lequel les cellules de bourgeon pancréatique sont des cellules humaines.

14. Procédé de prolifération de cellules PDX1-positives, comprenant l'étape de mise en culture de cellules PDX1-positives dans un milieu comprenant du KGF, de l'EGF, un inhibiteur de BMP, et un inhibiteur d'Akt.

15. Procédé selon la revendication 14, dans lequel les cellules sont mises en culture dans des conditions de culture adhérente.
